# EUROPEAN PATENT APPLICATION

(11) **EP 1 681 065 A1**
(43) Date of publication of application: **19.07.2006**
(21) Application number: 04799523.8
(22) Date of filing: 01.11.2004
(51) Int. Cl.: A61K 47/38, A61K 9/06, A61K 47/12, A61K 47/18, A61K 47/26, A61K 47/34, A61K 47/40

(54) **WATER-BASED COMPOSITION UNDERGOING REVERSIBLE THERMOGELATION**

(30) Priority: 31.10.2003 JP 2003371572
(71) Applicant: Wakamoto Pharmaceutical Co., Ltd., Chuo-ku, Tokyo 103-8330 (JP)
(72) Inventor: SUZUKI, Hidekazu; c/o WAKAMOTO PHARMACEUTICAL, Chuo-ku , Tokyo; 1038330 (JP)
(74) Representative: Vossius & Partner
(86) International application number: PCT/JP2004/016500
(87) International publication number: WO 2005/042026

(57) **Abstract**

The present invention relates to a reversibly heat gelable aqueous composition comprising a reversibly heat gelable aqueous composition according to conventional technique, to which a thixotropic property-increasing substance is added. The thixotropic property-increasing substance is preferably at least one member selected from the group consisting of sugar alcohol, lactose, carmellose or pharmaceutically acceptable salts thereof and cyclodextrin. This composition can be stored at room temperature and accordingly, it is quite convenient for users to carry about the same.

## Description

### Technical Field

The present invention relates to a reversibly heat-gelable aqueous composition having high thixotropy.

### Background Art

Japanese Patent No. 2,729,859 discloses a reversibly heat-gelable aqueous pharmaceutical composition prepared using methylcellulose, which can undergo gelation at a body temperature. This pharmaceutical composition is in a liquid state prior to administration thereof to a subject and therefore, it would be quite favorable for administration. On the other hand, it can be gelatinized at a body temperature and therefore, a viscosity thereof increases after administration thereof. Accordingly, the composition has such advantages that it can improve retention property of a drug at a drug-administered site of a subject and likewise improve bioavailability of the drug. An eye drop has been developed while making the most use of these characteristic properties and has already been put into practical use.

Moreover, Japanese Un-Examined Patent Publication (hereafter referred to as "JP-A") 2003-095924 discloses that this reversibly heat-gelable aqueous pharmaceutical composition is used as an artificial lacrimal fluid. This reversibly heat-gelable aqueous pharmaceutical composition permits an increase of the quantity of lacrimal fluid and the protection of a lacrimal oil layer as compared with conventional artificial lacrimal fluid and therefore, this composition has been recognized to be quite effective as an artificial lacrimal fluid.

In this connection, it is assumed that the reversibly heat-gelable aqueous pharmaceutical compositions disclosed in these patent documents are in general stored at a low temperature (for instance, 1 to 10°C). This is because if the reversibly heat-gelable aqueous pharmaceutical composition is stored at room temperature, in particular, in summer season, the composition gradually gelatinizes due to the action of environmental heat at room temperature to lose such characteristic properties that it is in a liquid state prior to administration and is quite favorable for administration.

In case of artificial lacrimal fluid, it is common that users in general carry them about and instill them several times a day. As discussed above, however, the reversibly heat-gelable aqueous pharmaceutical composition should be stored at a low temperature and accordingly, it is inappropriate to carry about the same. As described above, it is quite effective to use the reversibly heat-gelable aqueous pharmaceutical composition as an artificial lacrimal fluid from the viewpoint of its efficacy, but it is substantially disadvantageous from the viewpoint of storage thereof. For this reason, it has not yet been put into practical use.

### Disclosure of the Invention

Accordingly, it is an object of the present invention to provide a reversibly heat gelable aqueous pharmaceutical composition which may be carried about at room temperature, which can solve such a problem associated with conventional reversibly heat-gelable aqueous pharmaceutical composition that it is solidified through the gelatinization at room temperature and it cannot easily be administered.

The present invention has been completed based on such a finding that the foregoing object of the present invention can be achieved by the incorporation of a substance capable of increasing thixotropic property (hereafter referred to as "thixotropic property-increasing substance") into a reversibly heat-gelable aqueous composition and the present invention thus provides a reversibly heat-gelable aqueous composition detailed below:
1. A reversibly heat-gelable aqueous composition comprising methylcellulose and a thixotropic property-increasing substance.
2. The reversibly heat-gelable aqueous composition as set forth in the foregoing item 1, wherein the thixotropic property-increasing substance is at least one member selected from the group consisting of sugar alcohol, lactose, carmellose or pharmaceutically acceptable salts thereof and cyclodextrin.
3. The reversibly heat-gelable aqueous composition as set forth in the foregoing item 2, wherein the sugar alcohol is mannitol, xylitol or sorbitol.
4. The reversibly heat-gelable aqueous composition as set forth in the foregoing item 2, wherein the cyclodextrin is α -cyclodextrin, β -cyclodextrin or γ -cyclodextrin.
5. The reversibly heat-gelable aqueous composition as set forth in any one of the foregoing items 1 to 4, wherein the composition comprises at least one member selected from the group consisting of polyethylene glycol, amino acids or pharmaceutically acceptable salts thereof, and oxyacids or pharmaceutically acceptable salts thereof.
6. The reversibly heat-gelable aqueous composition as set forth in the foregoing item 5, wherein the oxyacid is citric acid or a pharmaceutically acceptable salt thereof.
7. The reversibly heat-gelable aqueous composition as set forth in any one of the foregoing items 1 to 6, wherein the composition comprises a drug.
8. The reversibly heat-gelable aqueous composition as set forth in the foregoing item 7, wherein the drug is at least one member selected from the group consisting of fungicidal agents, antibiotic agents, anti-allergic agents, anti-inflammatory agents, glaucoma-treating agents, vitamin preparations, immuno-suppressors, agents for preventing or treating diabetes, amino acids, cornea-protecting agents, agents for treating disorders of anterior epithelium of cornea, synthetic anti-bacterial agents, anti-malignant tumors, and anti-viral agents.
9. The reversibly heat-gelable aqueous composition as set forth in the foregoing item 7, wherein the drug is at least one member selected from the group consisting of amphotericin B, fluconazole, miconazole nitrate, sodium colistin methane-sulfonate, carbenicillin sodium, gentamicin sulfate, erythromycin, azithromycin, tobramycin, kanamycin, acitazanolast, levocabastine hydrochloride, ketotifen fumarate, sodium salt of cromoglicic acid, tranilast, betamethasone phosphate, dexamethasone, hydrocortisone, sodium diclofenac, pranoprofen, indomethacin, sodium bromfenac, meloxicam, lornoxicam, timolol maleate, bunazosin hydrochloride, latanoprost, nipradilol, carteolol hydrochloride, isopropyl unoproston, dorzolamide hydrochloride, flavin adenine dinucleotide, pyridoxal phosphate, cyanocobalamin, cyclosporin, tacrolimus, mycophenolic acid, amino-guanidine, epalrestat, aminoethyl sulfonic acid, sodium chondroitin sulfate, sodium hyaluronate, ciprofloxacin hydrochloride, lomefloxacin hydrochloride, ofloxacin, levofloxacin, pazufloxacin tosylate, gatifloxacin, moxifloxacin hydrochloride, mitomycin C, 5-fluorouracil, adriamycin, acyclbvir, gancyclovir, cidofovir, sorivudine, and trifluorothymidine.
10. The reversibly heat-gelable aqueous composition as set forth in any one of the foregoing items 7 to 9, wherein the composition is in the form of an injection, an oral formulation, an ear drop, a nasal drop, an eye drop or a liniment.
11. The reversibly heat-gelable aqueous composition as set forth in the foregoing item 10, wherein the composition is in the form of an eye drop.
12. The reversibly heat-gelable aqueous composition as set forth in any one of the foregoing items 1 to 11, wherein the composition is an artificial lacrimal fluid.

### Best Mode for Carrying Out the Invention

Thixotropy used herein means a kind of abnormal viscous behavior of a substance. More specifically, this means a behavior of a substance such that the substance in its gel state is transformed into its sol state having flowability by simply agitating or shaking the same, while the substance in its sol state can again return to its gel state when allowing the same to stand. The reversibly heat-gelable aqueous composition of the present invention is gelatinized by the application of heat, but it has thixotropic properties and therefore, the flowability of the gelatinized composition increases when lightly shaking the same and accordingly, it can easily be administered to a living body. The reversibly heat-gelable aqueous composition of the present invention administered to a living body is again easily gelatinized due to the action of a body temperature.

As substances capable of increasing thixotropic property of the reversibly heat-gelable aqueous composition of the present invention, there may be listed, for instance, sugar alcohol, lactose, carmellose or pharmaceutically acceptable salts thereof, or cyclodextrin. The amount of the substance capable of increasing thixotropic property of the composition of the present invention is not restricted to any specific range, inasmuch as the intended effect of the present invention can be ensured, but it in general ranges from 0.01 to 10% (w/v) and preferably 0.1 to 5% (w/v).

Examples of such sugar alcohols preferably used herein are mannitol, xylitol and sorbitol. Examples of the cyclodextrins are α -cyclodextrin, β -cyclodextrin and γ -cyclodextrin. Examples of pharmaceutically acceptable salts of carmellose include sodium and potassium salts.

As thixotropic property-increasing substance used in the present invention, particularly preferred are D-mannitol, D-sorbitol, xylitol and lactose. These substances may serve not only to increase thixotropic property of the reversibly heat-gelable aqueous composition, but also to reduce a gelling temperature of the heat-gelable aqueous composition.

It is desired that the reversibly heat-gelable aqueous composition of the present invention is in a liquid state in a low temperature area (for instance, an area at a temperature of not more than 15°C), while it can undergo gelation by the action of a body temperature of a mammal and therefore, a gelling temperature thereof preferably ranges from about 20 to about 40°C and more preferably 24 to 37°C.

A viscosity of methylcellulose (this will hereafter be abbreviated as simply "MC") used in the present invention is not likewise restricted to any particular level, but it is desirable to use MC having a viscosity, as determined at 20°C using a 2%(w/v) aqueous solution thereof, preferably ranging from 3 to 12000 mPa· sec. All sorts of MCs may be used alone or in any combination insofar as a viscosity thereof falls within the range specified above. A content of methoxyl groups in MC preferably ranges from 26 to 33% while taking into consideration the solubility of MC in water. Furthermore, MCs are classified on the basis of viscosity values of aqueous solutions thereof and a variety of products have been put on the market, for instance, those having nominal viscosity values of 4, 15, 25, 100, 400, 1500, and 8000 (in this respect, the numerical values each represent a viscosity value (mPa·sec) as determined at 20°C using an aqueous solution thereof having a concentration of 2% (w/v)) and they may easily be available. Preferably used herein are those each having a nominal viscosity ranging from 4 to 400 because of their easy handling properties. In this connection, the details of, for instance, the outline, specifications, applications and trade names thereof, are described in Dictionary of Pharmaceutical Excipients (edited by Japan Pharmaceutical Excipients Council published by THE YAKUJI NIPPO LIMITED).

In the present invention, MC may be used in any concentration inasmuch as it can show desired effects of the present invention, but the concentration thereof preferably ranges from 0.2 to 7% (w/v) and more preferably 1 to 4% (w/v). This is because if the concentration thereof is not more than 7% (w/v), the resulting composition has a viscosity preferably falling within the range which makes the handling of the composition easy, while if the concentration thereof is not less than 0.2% (w/v), the resulting composition is quite susceptible to gelation at a body temperature.

The polyethylene glycol (this will hereafter be abbreviated as "PEG") used in the composition of the present invention as auxiliary agent for gelation is commercially sold by Wako Pure Chemical Industries, Ltd. under the trade names of PEG-200, -300, -G00, -1000, -1540, -2000, -4000, -6000, -20000, -50000, -500000, -2000000 and -4000000; and NOF Corporation under the trade names of MACROGOL-200, -300, -400, -600, -1000, -1540, -4000, -6000, and -20000. Weight average molecular weight of PEG used in the present invention is not likewise restricted to any specific range, but it preferably ranges from 300 to 50000 and more preferably 1000 to 6000. This is because if the weight average molecular weight thereof is not less than 300, the resulting composition easily causes the desired liquid-gel phase transition, while if the weight average molecular weight thereof is not more than 50000, the composition in its liquid state does not have any undesirably high viscosity. Moreover, it is also possible to combine not less than 2 kinds of PEG products in such a manner that the weight average molecular weight of the resulting mixture should fall within the foregoing optimum range. In this connection, details of, for instance, the outline, specifications, applications and trade names thereof are described in Dictionary of Pharmaceutical Excipients (edited by Japan Pharmaceutical Excipients Council published by THE YAKUJI NIPPO LIMITED).

A content of PEG used as an auxiliary agent for gelation in the composition of the present invention preferably ranges from 0.1 to 13% (w/v) and more preferably 1 to 9% (w/v).

The composition of the present invention preferably contains an oxyacid or a pharmaceutically acceptable salt thereof as another auxiliary agent for gelation. Examples of oxyacids are citric acid, tartaric acid, malic acid and lactic acid. Moreover, the pharmaceutically acceptable salts of the oxyacids are, for instance, sodium and potassium salts. The composition of the present invention may comprise such an oxyacid or a pharmaceutically acceptable salt thereof in a content typically ranging from 0.01 to 7% (w/v) and preferably 0.05 to 4% (w/v).

The composition of the present invention preferably comprises, as an auxiliary agent for gelation, an amino acid or a pharmaceutically acceptable salt thereof. Examples of such amino acids usable herein are aspartic acid, glutamic acid, histidine, lysine, arginine, glycine, alanine, serine, proline and methionine. In addition, examples of pharmaceutically acceptable salts thereof are hydrochlorides, sulfates, sodium salts, and potassium salts. The composition of the present invention preferably comprises such an amino acid or a pharmaceutically acceptable salt thereof in a content usually ranging from 0.01 to 7% (w/v) and preferably 0.05 to 4% (w/v).

A preferred embodiment of the present invention provides a reversibly heat-gelable aqueous composition which comprises a thixotropic property-increasing substance in an amount ranging from 0.1 to 10% (w/v), MC in an amount ranging from 0.2 to 7% (w/v), PEG in an amount ranging from 0.1 to 13% (w/v) and an oxyacid or an amino acid in an amount ranging from 0.01 to 7% (w/v).

Another preferred embodiment of the present invention provides a reversibly heat-gelable aqueous composition which comprises a thixotropic property-increasing substance in an amount ranging from 0.1 to 5% (w/v), MC in an amount ranging from 0.2 to 7% (w/v), PEG in an amount ranging from 0.1 to 13% (w/v) and an oxyacid or an amino acid in an amount ranging from 0.05 to 4% (w/v).

Still another preferred embodiment of the present invention provides a reversibly heat-gelable aqueous composition which comprises a thixotropic property-increasing substance in an amount ranging from 0.1 to 5% (w/v), MC in an amount ranging from 1.0 to 4.0% (w/v), PEG in an amount ranging from 1 to 9.0% (w/v) and an oxyacid or an amino acid in an amount ranging from 0.05 to 4% (w/v).

The reversibly heat-gelable aqueous composition of the present invention may likewise comprise a drug. Examples of such drugs are fungicidal agents such as amphotericin B, fluconazole, and miconazole nitrate; antibiotic agents such as sodium colistin methane-sulfonate, carbenicillin sodium, gentamicin sulfate, erythromycin, azithromycin, tobramycin, and kanamycin,; anti-allergic agents such as acitazanolast, levocabastine hydrochloride, ketotifen fumarate, sodium salt of cromoglicic acid, and tranilast; anti-inflammatory agents such as betamethasone phosphate, dexamethasone, hydrocortisone, sodium diclofenac, pranoprofen, indomethacin, sodium bromfenac, meloxicam, and lornoxicam; glaucoma-treating agents such as timolol maleate, bunazosin hydrochloride, latanoprost, nipradilol, carteolol hydrochloride, isopropyl unoproston, and dorzolamide hydrochloride; vitamin preparations such as flavin adenine dinucleotide, pyridoxal phosphate, and cyanocobalamin; immuno-suppressors such as cyclosporin, tacrolimus, and mycophenolic acid; agents for preventing or treating diabetes such as amino-guanidine, and epalrestat; cornea-protecting agents such as aminoethyl sulfonic acid, amino acids, and sodium chondroitin sulfate; agents for treating disorders of anterior epithelium of cornea such as sodium hyaluronate; synthetic anti-bacterial agents such as ciprofloxacin hydrochloride, lomefloxacin hydrochloride, ofloxacin, levofloxacin, pazufloxacin tosylate, gatifloxacin, and moxifloxacin hydrochloride; anti-malignant tumor agents such as mitomycin C, 5-fluorouracil, and adriamycin; and anti-viral agents such as acyclovir, gancyclovir, cidofovir, sorivudine, and triffuorothymidine. An amount of these drugs to be incorporated into the composition is not restricted to any specific one, insofar as they can show the desired efficacy thereof.

A pH value of the reversibly heat-gelable aqueous composition of the present invention is in general controlled to a level ranging from 4 to 10 and, in particular, it is preferred to control the pH value from 6 to 8. A variety of pH adjusters currently used such as acids and bases may be added to control a pH value of the reversibly heat-gelable aqueous composition of the present invention. Examples of such acids are ascorbic acid, hydrochloric acid, gluconic acid, acetic acid, lactic acid, boric acid, phosphoric acid, sulfuric acid, and citric acid. Examples of bases include potassium hydroxide, calcium hydroxide, sodium hydroxide, magnesium hydroxide, monoethanolamine, diethanolamine and triethanolamine. In addition to the foregoing, examples of other pH adjusters are amino acids such as glycine, histidine and ε -amino-caproic acid.

When preparing a reversibly heat-gelable aqueous composition according to the present invention, other pharmaceutically acceptable additives such as an isotonizing agent, a solubilizing agent, a preservative and/or an antiseptic can, if necessary, be incorporated into the composition in an amount which does not adversely affect the intended effects of the present invention. Examples of such isotonizing agent include saccharides such as glucose; propylene glycol, glycerin, sodium chloride and potassium chloride. Examples of such solubilizing agents are polysorbate 80 and polyoxyethylene-hardened castor oil. Examples of the foregoing preservatives include cationic soaps such as benzalkonium chloride, benzethonium chloride and chlorhexidine gluconate; parabens such as methyl p-hydroxy benzoate, propyl p-hyclioxy benzoate and butyl p-hydroxy benzoate; alcohols such as chloro-butanol, phenylethyl alcohol and benzyl alcohol, organic acids such as sodium dehydroacetate, sorbic acid and potassium sorbate and salts thereof. In addition, examples of other additives are thickening agents such as hydroxyethyl cellulose, poly(vinyl pyrrolidone), poly(vinyl alcohol), propylene glycol, diethylene glycol or sodium polyacrylate; and stabilizers such as EDTA (ethylenediamine-tetraacetic acid) and pharmaceutically acceptable salts thereof, tocopherol and derivatives thereof and sodium sulfite.

The following is an example of the method for the preparation of the reversibly heat-gelable aqueous composition of the present invention. First, MC and PEG are dispersed in hot water maintained at a temperature of not less than 70 °C and then ice-cooled. To the resulting dispersion, there are added a thixotropic property-increasing substance, an oxyacid or an amino acid, a drug, and additives and they are sufficiently admixed together to thus give a solution. A pH value thereof is controlled and sterilized and purified water is added to a desired volume to thus prepare a reversibly heat-gelable aqueous composition of the present invention. The reversibly heat-gelable aqueous composition of the present invention thus prepared is sterilized by filtration through a membrane filter and then packaged in a container such as a glass ampule.

The reversibly heat-gelable aqueous composition of the present invention possesses strong thixotropic property. Accordingly, even if the heat-gelable aqueous composition is gelatinized at room temperature, the composition can easily restore its liquid state by lightly shaking the same in the gel state and can thus easily be administered to a subject. Contrary to this, it is essential for reversibly heat-gelable aqueous composition according to conventional technique to store the same at a cold place. However, the reversibly heat-gelable aqueous composition of the present invention is quite advantageous in that it can be stored at room temperature and accordingly, users may always carry about the same with themselves.

The reversibly heat-gelable aqueous composition of the present invention can be used as an artificial lacrimal fluid, while making the most use of the characteristic properties thereof, or it may likewise be used in the form of, for instance, an injection, an oral formulation, an ear drop, a nasal drop, an eye drop or a liniment.

The present invention will hereafter be described in more specifically with reference to the following Examples.

### Example 1

There were mixed predetermined amounts of methylcellulose (METOLOSE (registered trade mark) SM-4 available from Shin-Etsu Chemical Co., Ltd.) and polyethylene glycol (MACROGOL 4000 available from NOF Corporation), followed by addition of sterilized and purified water heated to 85 °C to the resulting mixture, and stirring thereof to thus give a dispersion. After confirming the formation of a uniformly dispersed mixture, the dispersion was ice-cooled with stirring. After confirming the formation of an entirely transparent dispersion, there were gradually added, to the dispersion, predetermined amounts of sodium citrate and a thixotropic property-increasing substance used in the present invention (D-mannitol, D-sorbitol, xylitol, lactose, sodium salt of carmellose, a -cyclodextrin, β -cyclodextrin and γ -cyclodextrin). After the dissolution thereof, the resulting product was uniformly mixed together. Further, a pH value thereof was adjusted to 7.0 with a IN NaOH solution or a IN HCl solution and then sterilized and purified water was added to a predetermined volume (100 mL) to thus give a reversibly heat-gelable aqueous composition of the present invention.

The same procedures used above were repeated except for omitting the addition of any thixotropic property-increasing substance to thus give a separate reversibly heat-gelable aqueous composition as a comparative example.

### [Test Examples]

The reversibly heat-gelable aqueous compositions prepared in the foregoing Example were inspected for the gelling temperature and thixotropic property.

### • Gelling Temperature:

To the BL adapter of a Brookfield type viscometer (B-type viscometer) available from TOKIMEC INC., there was added 25 mL of each reversibly heat-gelable aqueous composition prepared above and the adapter together with the container therefor was ice-cooled for 10 minutes. The adapter was then attached to the B-type viscometer, the BL adapter portion of the resulting assembly was dipped in a water bath whose temperature had been controlled to a desired level and the adapter was maintained under such conditions for 3 minutes. Thereafter, a synchronous electric motor for the B-type viscometer was switched on (the rotor initiated the rotational motion), followed by the reading off of the indicated value (viscosity value as expressed in the unit of milli-Pascal second (mPa . s)) observed after 2 minutes. Each indicated value was defined to be the viscosity of the composition at that temperature. The viscosity values were determined starting from the temperature of 20°C up to 40°C at intervals of 2°C. The gelling temperature was defined to be the measuring temperature immediately before that at which the composition showed a viscosity increase of not less than 0.3 mPa · s.

### Thixotropic Property

Each reversibly heat-gelable aqueous composition (5 mL) prepared above was packed in a commercially available eye drop bottle (having an inner diameter of 17 mm and a height of 35 mm) made of a plastic material. The bottle was maintained at 40°C for 4 hours to thus gelatinize the reversibly heat-gelable aqueous composition. Immediately thereafter, the content of the eye drop bottle was stirred by gently making the bottle turn over 7 times. At this stage, if the gel disintegrated and accordingly, the reversibly heat-gelable aqueous composition was easily discharged from the drop bottle, the composition was judged to have thixotropic property. On the other hand, if the gel did not disintegrate or the reversibly heat-gelable aqueous composition was not easily discharged from the eye drop bottle even if the gel disintegrated after the stirring of the content of the bottle by turning over the bottle, the composition was judged not to have thixotropic property.

The following Table 1 shows the details of the prescriptions of the reversibly heat-gelable aqueous compositions thus prepared, and gelling temperature and thixotropic property thereof.

**Table 1: Gelling Temperature and Thixotropic Property of Reversibly Heat-Gelable Aqueous Composition of the Present Invention**

| Prescription No.* | 1 | 2 | 3 | 4 | 5 | 6 | 7 | 8 | 9* |
|---|---|---|---|---|---|---|---|---|---|
| SM-4(w/v%) | 1.2 | 1.2 | 1.2 | 1.2 | 1.2 | 1.2 | 1.2 | 1.2 | 1.2 |
| MACROGOL 4000 (w/v%) | 2.0 | 2.0 | 2.0 | 2.0 | 2.0 | 2.0 | 2.0 | 2.0 | 2.0 |
| Sodium Citrate (w/v%) | 3.5 | 3.5 | 3.5 | 3.5 | 3.5 | 3.5 | 3.5 | 3.5 | 3.5 |
| D-Mannitol (w/v%) | 0.8 | -- | -- | -- | -- | -- | -- | -- | -- |
| D-Sorbitol (w/v%) | -- | 0.8 | -- | -- | -- | -- | -- | -- | -- |
| Xylitol (w/v%) | -- | -- | 0.8 | -- | -- | -- | -- | -- | -- |
| Lactose (w/v%) | -- | -- | -- | 0.8 | -- | -- | -- | -- | -- |
| Sodium Carmellose (w/v%) | -- | -- | -- | -- | 0.8 | -- | -- | -- | -- |
| α - Cyclodextrin (w/v%) | -- | -- | -- | -- | -- | 0.8 - | -- | -- | -- |
| β - Cyclodextrin (w/v%) | -- | -- | -- | -- | -- | -- | 0.8 | -- | -- |
| γ -Cyclodextrin (w/v%) | -- | -- | -- | -- | -- | -- | -- | 0.8 - | -- |
| NaOH or HCl | As much as suffices | | | | | | | | |
| Water | As much as suffices | | | | | | | | |
| pH | 7.0 | 7.0 | 7.0 | 7.0 | 7.0 | 7.0 | 7.0 | 7.0 | 7.0 |
| Gelling Temp. (°C) | 28 | 28 | 28 | 30 | 32 | 32 | 32 | 32 | 32 |
| Thixotropic Property | yes | yes | yes | yes | yes | yes | yes | yes | no |

| | | | | | | | | | |
|---|---|---|---|---|---|---|---|---|---|
| *: Prescription No.9 is Comparative Example. | | | | | | | | | |

The data listed in Table 1 clearly indicate that all of the reversibly heat-gelable aqueous compositions according to the present invention having the foregoing different prescriptions can be gelatinized at a temperature near a body temperature and that all of them possess thixotropic property. On the other hand, the composition of Comparative Example which is free of any thixotropic property-increasing substance can be gelatinized at a temperature near a body temperature, but it holds its hard gel state even when stirred by turning over the container thereof and in other words, it does not show thixotropic property.

Moreover, the data listed in Table 1 likewise indicate that when incorporating, into the composition, D-mannitol, D-sorbitol, xylitol or lactose as a thixotropic property-increasing substance, the resulting compositions have reduced gelling temperatures and therefore, they can more easily be gelatinized at a body temperature.

### Example 2

There were mixed predetermined amounts of methylcellulose (SM-4) and polyethylene glycol (MACROGOL 4000), followed by addition of sterilized and purified water heated to 85°C to the resulting mixture, and stirring thereof to thus give a dispersion. After confirming the formation of a uniformly dispersed mixture, the dispersion was ice-cooled with stirring. After confirming the formation of an entirely transparent dispersion, there were gradually added, to the dispersion, predetermined amounts of sodium citrate, glycine, D-mannitol, aminoethyl sulfonic acid, sodium chondroitin sulfate, and benzalkonium chloride. After the dissolution thereof, the resulting product was uniformly mixed together. Further, a pH value thereof was adjusted to 7.4 with a IN HCl solution and then sterilized and purified water was added to a predetermined volume (100 mL) to thus give a reversibly heat-gelable aqueous composition of the present invention.

The following Table 2 shows the details of the prescriptions of the reversibly heat-gelable aqueous compositions thus prepared, and gelling temperature and thixotropic property thereof.

**Table 2: Gelling Temperature and Thixotropic Property of Reversibly Heat-Gelable Aqueous Composition of the Present Invention**

| Prescription No. | 10 | 11 | 12 |
|---|---|---|---|
| Aminoethyl sulfonic acid (w/v%) | 0.1 | -- | -- |
| Sodium chondroitin sulfate (w/v%) | -- | 0.1 1 | 0.1 |
| SM-4 (w/v%) | 1.0 | 1.3 | 4.0 |
| MACROGOL 4000 (w/v%) | 2.0 | 2.0 | 4.0 |
| Sodium citrate (w/v%) | 2.0 | 1.5 | -- |
| Glycine (w/v%) | -- | -- | 1.8 |
| D-Mannitol (w/v%) | 1.5 | 2.0 | 1.0 |
| Benzalkonium chloride (w/v%) | 0.005 | 0.005 | 0.005 |
| HCl | As much as suffices | | |
| Water | As much as suffices | | |
| pH | 7.4 | 7.4 | 7.4 |
| Gelling temperature (°C) | 32 | 32 | 34 |
| Thixotropic Property | yes | yes | yes |

### Example 3

There were mixed predetermined amounts of methylcellulose (SM-4) and D-mannitol as a thixotropic property-increasing substance, followed by addition of sterilized and purified water heated to 85°C to the resulting mixture, and stirring thereof to thus give a dispersion. After confirming the formation of a uniformly dispersed mixture, the dispersion was ice-cooled with stirring. After confirming the formation of an entirely transparent dispersion, there were gradually added, to the dispersion, predetermined amounts of sodium citrate, sodium tartrate or sodium glutamate depending on the prescriptions. After the dissolution thereof, the resulting product was uniformly mixed together. Further, the pH value thereof was adjusted to 7.4 with a IN NaOH solution or a IN HCl solution and then sterilized and purified water was added to a predetermined volume (100 mL) to thus give a reversibly heat-gelable aqueous composition of the present invention.

Then the reversibly heat-gelable aqueous composition thus prepared was inspected or evaluated for gelling temperature and thixotropic property

The following Table 3 shows the details of the prescriptions of the reversibly heat-gelable aqueous compositions thus prepared, and gelling temperature and thixotropic property thereof.

**Table 3: Gelling Temperature and Thixotropic Property of Reversibly Heat-Gelable Aqueous Composition of the Present Invention**

| Prescription No. | 13 | 14* | 15 | 16* | 17 | 18* | 19 | 20* |
|---|---|---|---|---|---|---|---|---|
| SM-4 (w/v%) | 6.0 | 6.0 | 1.5 | 1.5 | 1.5 | 1.5 | 1.5 | 1.5 |
| D-Mannitol(w/v%) | 1.5 | -- | 1.5 | -- | 1.5 | -- | 1.5 | -- |
| Sodium citrate (w/v%) | -- | -- | 3.0 | 3.0 | -- | -- | -- | -- |
| Sodium tartrate (w/v%) | -- | -- | -- | -- | 3.2 | 3.2 | -- | -- |
| Sodium glutamate (w/v%) | -- | -- | -- | -- | -- | -- | 3.5 | 3.5 |
| NaOH or HCl | As much as suffices | | | | | | | |
| Water | As much as suffices | | | | | | | |
| pH | 7.4 | 7.4 | 7.4 | 7.4 | 7.4 | 7.4 | 7.4 | 7.4 |
| Gelling Temperature (°C) | 32 | 34 | 30 | 32 | 32 | 34 | 34 | 34 |
| Thixotropic Property | yes | no | yes | no | yes | no | yes | no |

| | | | | | | | | |
|---|---|---|---|---|---|---|---|---|
| *: Prescription Nos. 14, 16, 18 and 20 are Comparative Examples. | | | | | | | | |

The data listed in Table 3 clearly indicate that all of the reversibly heat-gelable aqueous compositions according to the present invention having the foregoing different prescriptions can be gelatinized near a body temperature and that all of them possess thixotropic property. On the other hand, the compositions of Comparative Examples which are free of any thixotropic property-increasing substance can be gelatinized near a body temperature, but they hold their hard gel states even when stirred by turning over the containers thereof and in other words, they do not show thixotropic property.

### Example 4 (Injection)

There were mixed 11 g of methylcellulose (SM-4), 20 g of polyethylene glycol (MACROGOL 4000) and 15 g of D-mannitol, followed by addition of sterilized and purified water (800 mL) heated to 85°C to the resulting mixture, and stirring thereof to thus give a dispersion. After confirming the formation of a uniformly dispersed mixture, the dispersion was ice-cooled with stirring. After confirming the formation of an entirely transparent dispersion, there were gradually added, to the dispersion, 20 g of sodium citrate and 1.0 g of levofloxacin. After the dissolution thereof, the resulting product was uniformly mixed together. Further, a pH value thereof was adjusted to 7.4 with a 1N HCl solution and then sterilized and purified water was added to a final volume of 1000 mL to thus give a reversibly heat-gelable aqueous composition of the present invention.

The resulting aqueous composition was filtered through a membrane filter, packed in a glass ampule having a volume of 5 mL and then the ampule was sealed through melting to thus give an injection.

### Example 5 (Nasal Drop)

There were mixed 14 g of methylcellulose (SM-4), 20 g of polyethylene glycol (MACROGOL 4000) and 15 g of D-mannitol, followed by addition of sterilized and purified water (800 mL) heated to 85°C to the resulting mixture, and stirring thereof to thus give a dispersion. After confirming the formation of a uniformly dispersed mixture, the dispersion was ice-cooled with stirring. After confirming the formation of an entirely transparent dispersion, there were gradually added, to the dispersion, 20 g of sodium citrate and 3.0 g of levofloxacin. After the dissolution thereof, the resulting product was uniformly mixed together. Further, a pH value thereof was adjusted to 7.4 with a IN HCl solution and then sterilized and purified water was added to a final volume of 1000 mL to thus give a reversibly heat-gelable aqueous composition of the present invention.

The resulting aqueous composition was filtered through a membrane filter and then packed in a nasal drop container made of a plastic to thus give a nasal drop.

### Example 6 (Ear Drop)

The reversibly heat-gelable aqueous composition of the present invention prepared in Example 5 was filtered through a membrane filter and then packed in an ear drop container made of a plastic to thus give an ear drop.

### Example 7 (Liniment)

The reversibly heat-gelable aqueous composition of the present invention prepared in Example 5 was filtered through a membrane filter and then packed in a plastic container to thus give a liniment.

### Example 8 (Oral Formulation)

The reversibly heat-gelable aqueous composition of the present invention prepared in Example 4 was filtered through a membrane filter and then packed in a glass container to thus give an oral formulation.

### Example 9 (Eye Drop)

The reversibly heat-gelable aqueous composition of the present invention prepared in Example 5 was filtered through a membrane filter and then packed in an eye drop container made of a plastic having a volume of 5 mL to thus give an eye drop.

### Example 10 (Artificial Lacrimal Fluid)

There were mixed 10 g of methylcellulose (SM-4), 20 g of polyethylene glycol (MACROGOL 4000) and 15 g of D-mannitol, followed by addition of sterilized and purified water (800 mL) heated to 85°C to the resulting mixture, and stirring thereof to thus give a dispersion. After confirming the formation of a uniformly dispersed mixture, the dispersion was ice-cooled with stirring. After confirming the formation of an entirely transparent dispersion, there were gradually added, to the dispersion, 20 g of sodium citrate and 5 g of aminoethyl sulfonic acid. After the dissolution thereof, the resulting product was uniformly mixed together. Further, a pH value thereof was adjusted to 7.4 with a IN HCl solution and then sterilized and purified water was added to a final volume of 1000 mL to thus give a reversibly heat-gelable aqueous composition of the present invention.

The resulting aqueous composition was filtered through a membrane filter and then packed in an eye drop container made of a plastic having a volume of 5 mL to thus give an artificial lacrimal fluid.

### Example 11 (Artificial Lacrimal Fluid)

There were mixed 13 g of methylcellulose (SM-4), 20 g of polyethylene glycol (MACROGOL 4000) and 10 g of D-mannitol, followed by addition of sterilized and purified water (800 mL) heated to 85°C to the resulting mixture, and stirring thereof to thus give a dispersion. After confirming the formation of a uniformly dispersed mixture, the dispersion was ice-cooled with stirring. After confirming the formation of an entirely transparent dispersion, there were gradually added, to the dispersion, 20 g of sodium citrate and 2 g of NaCl. After the dissolution thereof, the resulting product was uniformly mixed together. Further, a pH value thereof was adjusted to 7.4 with a IN HCl solution and then sterilized and purified water was added to a final volume of 1000 mL to thus give a reversibly heat-gelable aqueous composition of the present invention.

The resulting aqueous composition was filtered through a membrane filter and then packed in an eye drop container made of a plastic having a volume of 5 mL to thus give an artificial lacrimal fluid.

### Example 12 (Artificial Lacrimal Fluid)

There were mixed 11 g of methylcellulose (SM-4), 20 g of polyethylene glycol (MACROGOL 4000) and 15 g of D-mannitol, followed by addition of sterilized and purified water (800 mL) heated to 85°C to the resulting mixture, and stirring of the same to thus give a dispersion. After confirming the formation of a uniformly dispersed mixture, the dispersion was ice-cooled with stirring. After confirming the formation of an entirely transparent dispersion, there was gradually added, to the dispersion, 22 g of sodium citrate. After the dissolution thereof, the resulting product was uniformly mixed together. Further, a pH value thereof was adjusted to 7.4 with a 1N HCl solution and then sterilized and purified water was added to a final volume of 1000 mL to thus give a reversibly heat-gelable aqueous composition of the present invention.

The resulting aqueous composition was filtered through a membrane filter and then packed in an eye drop container made of a plastic having a volume of 5 mL to thus give an artificial lacrimal fluid.

### Industrial Applicability

The reversibly heat-gelable aqueous composition according to the present invention possesses strong thixotropic property. Accordingly, even if the heat-gelable aqueous composition undergoes gelation at room temperature, the composition in its gel state can easily be fluidized by shaking the same. The composition can be carried about at room temperature and can be used as an artificial lacrimal fluid while making the most use of the characteristic properties. In addition, the composition can likewise be used in the form of, for instance, an injection, an oral formulation, an ear drop, a nasal drop, an eye drop or a liniment.

## Claims

1. A reversibly heat-gelable aqueous composition comprising methylcellulose and a thixotropic property-increasing substance.

2. The reversibly heat-gelable aqueous composition as set forth in claim 1, wherein the thixotropic property-increasing substance is at least one member selected from the group consisting of sugar alcohol, lactose, carmellose or pharmaceutically acceptable salts thereof and cyclodextrin.

3. The reversibly heat-gelable aqueous composition as set forth in claim 2, wherein the sugar alcohol is mannitol, xylitol or sorbitol.

4. The reversibly heat-gelable aqueous composition as set forth in claim 2, wherein the cyclodextrin is a cyclodextrin, β -cyclodextrin or γ -cyclodextrin.

5. The reversibly heat-gelable aqueous composition as set forth in any one of claims 1 to 4, wherein the composition comprises at least one member selected from the group consisting of polyethylene glycol, amino acids or pharmaceutically acceptable salts thereof, and oxyacids or pharmaceutically acceptable salts thereof.

6. The reversibly heat-gelable aqueous composition as set forth in claim 5, wherein the oxyacid is citric acid or a pharmaceutically acceptable salt thereof.

7. The reversibly heat-gelable aqueous composition as set forth in any one of claims 1 to 6, wherein the composition comprises a drug.

8. The reversibly heat-gelable aqueous composition as set forth in claim 7, wherein the drug is at least one member selected from the group consisting of fungicidal agents, antibiotic agents, anti-allergic agents, anti-inflammatory agents, glaucoma-treating agents, vitamin preparations, immuno-suppressors, agents for preventing or treating diabetes, amino acids, cornea-protecting agents, agents for treating disorders of anterior epithelium of cornea, synthetic anti-bacterial agents, anti-malignant tumors, and anti-viral agents.

9. The reversibly heat-gelable aqueous composition as set forth in claim 7, wherein the drug is at least one member selected from the group consisting of amphotericin B, fluconazole, miconazole nitrate, sodium colistin methane-sulfonate, carbenicillin sodium, gentamicin sulfate, erythromycin, azithromycin, tobramycin, kanamycin, acitazanolast, levocabastine hydrochloride, ketotifen fumarate, sodium salt of cromoglicic acid, tranilast, betamethasone phosphate, dexamethasone, hydrocortisone, sodium diclofenac, pranoprofen, indomethacin, sodium bromfenac, meloxicam, lornoxicam, timolol maleate, bunazosin hydrochloride, latanoprost, nipradilol, carteolol hydrochloride, isopropyl unoproston, dorzolamide hydrochloride, flavin adenine dinucleotide, pyridoxal phosphate, cyanocobalamin, cyclosporin, tacrolimus, mycophenolic acid, amino-guanidine, epalrestat, aminoethyl sulfonic acid, sodium chondroitin sulfate, sodium hyaluronate, ciprofloxacin hydrochloride, lomefloxacin hydrochloride, ofloxacin, levofloxacin, pazufloxacin tosylate, gatifloxacin, moxifloxacin hydrochloride, mitomycin C, 5-fluorouracil, adriamycin, acyclovir, gancyclovir, cidofovir, sorivudine, and trifluorothymidine.

10. The reversibly heat-gelable aqueous composition as set forth in any one of claims 7 to 9, wherein the composition is in the form of an injection, an oral formulation, an ear drop, a nasal drop, an eye drop or a liniment.

11. The reversibly heat-gelable aqueous composition as set forth in claim 10, wherein the composition is in the form of an eye drop.

12. The reversibly heat-gelable aqueous composition as set forth in any one of claims 1 to 11, wherein the composition is an artificial lacrimal fluid.
